# EUROPEAN PATENT APPLICATION

(11) **EP 4 691 568 A2**
(43) Date of publication of application: **11.02.2026**
(21) Application number: 25225699.5
(22) Date of filing: 07.06.2018
(51) Int. Cl.: A61P 37/08

(54) **LOW-DOSE BRIMONIDINE COMBINATIONS AND USES THEREOF**

(30) Priority: 08.06.2017 US 201762516931 P; 24.01.2018 US 201862621082 P
(62) Divisional of application: 18813969.5
(71) Applicant: Eye Therapies, LLC, Dana Point, CA 92654 (US)
(72) Inventor: HORN, Gerald, Deerfield, 60015 (US)
(74) Representative: Elkington and Fife LLP

(57) **Abstract**

The present invention is directed to compositions containing low-dose brimonidine and either a second glaucoma drug, an anti-histamine or non-steroidal anti-inflammatory drug. The present invention is further directed to methods of treating glaucoma or allergies or reducing inflammation by administering compositions of the present invention.

## Description

### FIELD OF THE INVENTION

The present invention is related to compositions containing low-dose brimonidine and either a second glaucoma drug, an anti-histamine or a non-steroidal anti-inflammatory drug. The present invention is further related to methods of treating glaucoma or allergies or reducing inflammation by administering compositions of the present invention.

### BACKGROUND OF THE INVENTION

Glaucoma is a multifactorial disease which encompasses a spectrum ranging from elevated intraocular pressure ("IOP") to reduced vascular perfusion of the optic nerve.

While many factors have been implicated as contributing causes of glaucoma, currently existing treatments for glaucoma have limited effectiveness in lowering IOP and/or are accompanied by a number of side effects, such as fatigue, sedation, lid allergy, topical allergy, and/or redness. More than 10% of glaucoma patients suffer the stigma of hyperemia (i.e. eye redness). Because of the side effects, an additional major problem in glaucoma therapy is patient compliance in taking medications as prescribed.

Over 40% of glaucoma patients require two or more drugs for satisfactory control of their intraocular pressure. Of these, the prostaglandins/prostanoids, including Xalatan^{®} (latanoprost; Xalatan is a registered trademark of Pfizer Health AB), Travatan^{®} (travoprost; Travatan is a registered trademark of Novartis AG) and Lumigan^{®} (bimatoprost; Lumigan is a registered trademark of Allergan, Inc.), are the leading drugs due to their profound reduction of IOP, typically above 30% in ocular hypertensive eyes (21 mm Hg or greater), and long duration improvement in uveoscleral outflow. To have the greatest effect, the two drugs should have different mechanisms of action.

Brimonidine, a known alpha-2 (α-2) adrenergic receptor agonist, is currently available under the tradenames Alphagan^{®} (Alphagan is a registered trademark of Allergan, Inc.) and in the form of 0.1%, 1.5% and 0.2% brimonidine tartrate. Brimonidine typically causes moderate peak IOP reduction of about 20-25% in ocular hypertensive eyes and 6-18% in normotensive eyes (less than 21 mm Hg). Its peak effect is within 2 hours of instillation, its duration of effect is typically less than 12 hours, and its moderate efficacy usually requires dosing of 2-3 times a day. It is one of the leading secondary drugs, with a mechanism of action of aqueous suppression that complements the prostaglandin/prostanoids uveal scleral outflow enhancement for significant additive benefit, but about equal to other second line glaucoma drugs such as beta-blockers and carbonic anhydrase inhibitors. 0.2% brimonidine has been found to provide an additional 1 to 2 mm Hg reduction in IOP when combined with a beta-blocker or carbonic anhydrase inhibitors and particularly with a prostaglandin.

However, 0.15-0.2% brimonidine may induce substantial local side effects in 10-25% of users, such as conjunctival hyperemia (i.e. redness), blepharitis, allergy, conjunctival edema, conjunctival follicles, foreign body sensation, burning, or blurring. These side effects were only modestly improved by recent brimonidine formulations, resulting in somewhat reduced concentrations with increased intraocular absorption at more alkaline pH (Alphagan^{®}). In general, α-2 agonists, including brimonidine, its predecessor clonidine and the more selective dexmedetomidine, induce substantial systemic effects if absorbed into the circulation, and are specifically known to increase fatigue, decrease blood pressure (i.e. hypotension) and lower the heart rate (i.e. bradycardia). Brimonidine, due to its relative alpha 2/ alpha 1 specificity (about a 900:1 ratio) still introduces alpha 1 agonist activity intraocularly, where any induced ischemia long term can potentially accelerate optic nerve loss.

Thus, there is a need in the art for a combination glaucoma treatment that reduces side effects while maintaining at least an additive effect.

Allergic diseases are a major health problem worldwide. Furthermore, the prevalence of allergic disease is increasing in most countries. Two major forms of allergies include allergic rhinitis and ocular allergies. Allergic reactions occur, in part, when the immune system reacts to the presence of an allergen and produces histamine. Histamine then causes the major symptoms of allergies including a "runny" or "itchy" nose, sneezing, "watery" eyes, itchy throat, etc.

In allergic rhinitis, the allergic reaction occurs in the nose. Histamine released in response to the reaction causes local vascular dilation, with resultant increased capillary pressure as well as increased capillary permeability. Both of these effects cause rapid fluid leakage into the tissues of the nose and the nasal linings become swollen and secretory.

Ocular allergies or allergic conjunctivitis often accompanies allergic rhinitis in response to the release of histamines. Ocular signs and symptoms of allergies include itching, redness, swelling, tearing, burning and stinging.

Ocular allergies are often treated with eye drops including Opcon-A^{®} (0.02675% naphazoline hydrochloride and 0.315% pheniramine maleate; Opcon A is a registered trademark of Bausch & Lomb Incorporated), Naphcon-A^{®} (0.025% naphazoline hydrochloride and 0.3% pheniramine maleate; Naphcon-A is a registered trademark of Alcon Research Ltd), and Vasocon^{®} (0.05% naphazoline hydrochloride and 0.5% antazoline phosphate; Vasocon is a registered trademark of Novartis AG) and Albalon^{™} (0.05% naphazoline hydrochloride, Albalon is available from Allergan Pharmaceuticals).

However, many of the anti-histamines prescribed or administered to treat ocular allergies and or allergic rhinitis treat histamine induced itching and discomfort but do not fully alleviate the attendant symptoms including particularly swelling secondary to vascular leakage and hyperemia (eye redness) resulting in both remaining discomfort and an unhealthy appearance. Thus, there is a further need in the art for a combination allergy treatment that leads to a greater alleviation of attendant symptoms including eye redness.

### SUMMARY OF THE INVENTION

In one embodiment, the present invention is directed to an ophthalmological composition for the treatment of glaucoma comprising brimonidine at a concentration from about 0.01% to about 0.050% w/v and a second glaucoma drug.

In another embodiment, the present invention is directed to a method of treating glaucoma comprising administering a composition comprising brimonidine at a concentration from about 0.01% to about 0.050% w/v and a second glaucoma drug to a subject in need thereof.

In another embodiment, the present invention is directed to a method of treating glaucoma, wherein the method further provides eye redness reduction.

In another embodiment, the present invention is directed to a method of treating glaucoma, wherein the method further provides eye whitening.

In another embodiment, the present invention is directed to a method of treating glaucoma, wherein the method further provides conjunctival swelling reduction as well as hyperemia reduction and eye whitening.

In one embodiment, the present invention is directed to an ophthalmological composition for the treatment of allergies comprising brimonidine at a concentration from about 0.01% to about 0.050% w/v and a histamine antagonist.

In another embodiment, the present invention is directed to a method of treating allergies comprising administering a composition comprising brimonidine at a concentration from about 0.005% to about 0.050% w/v and a histamine antagonist to a subject in need thereof.

In another embodiment, the present invention is directed to a method of treating allergies, wherein the method further provides eye whitening and or reduced inflammation.

In another embodiment, the present invention is directed to an ophthalmological composition for the reduction of inflammation comprising from about 0.005% to about 0.050% w/v brimonidine and an effective amount of a nonsteroidal anti-inflammatory drug ("NSAID").

In another embodiment, the present invention is directed to a method of reducing inflammation, comprising administering a composition comprising brimonidine at a concentration from about 0.005% to about 0.050% w/v and an NSAID to a subject in need thereof.

### BRIEF DESCRIPTION OF THE DRAWINGS

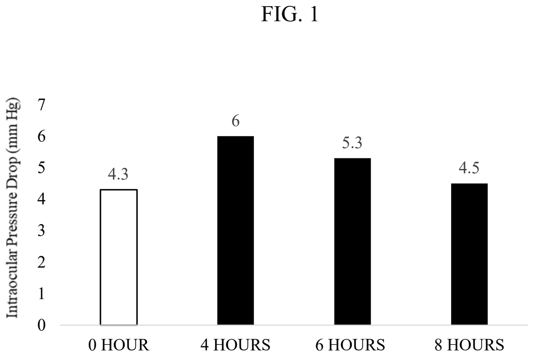

Figure 1- a prophetic example of IOP reduction after administration of 0.005% latanoprost (0 hours, white bar), versus IOP reduction after administration of 0.03% brimonidine and 0.005% latanoprost (4, 6 and 8 hours, black bar).

### DETAILED DESCRIPTION OF THE INVENTION

It is a discovery of the present invention that postcapillary venular leakage may be reduced selectively by low dose brimonidine over alpha 1 vasoconstrictors. Not wishing to be held to particular theory, it is believed alpha 1 agonists constrict larger vessels such as arterioles to a much greater extent than low dose brimonidine. The constriction of the larger vessels creates ischemia, and with repeated use, inflammation and rebound hyperemia. The leakage in postcapillary venules has been shown to be related to transient loss of vascular endothelial VE cadherin protein resulting in gap junctions with leakage along the postcapillary venular cell wall. However, constriction of small vessels and particularly said postcapillary venules without ischemia by low dose brimonidine is surprisingly found to reduce such leakage without the ischemia of tetrahydrozaline, oxymetazoline, or other alpha 1 agonist constrictors, alleviating redness and clinical sequelae of swelling and redness otherwise not as completely alleviated making the combination of low dose brimonidine with an anti-histamine particularly effective.

When combined with glaucoma drugs, such as particularly but not limited to prostaglandins/prostanoids, surface inflammation, swelling and hyperemia is reduced. Further IOP is further reduced. The present invention discovers lower concentrations of brimonidine provide effective second drug IOP lowering benefits but also potentially reduce optic nerve ischemia, without the attendant alpha 1 agonist activity of higher concentrations of brimonidine.

The term "brimonidine" encompasses, without limitation, brimonidine salts and other derivatives, and specifically includes, but is not limited to, brimonidine tartrate, 5-bromo-6-(2-imidazolin-2-ylamino)quinoxaline D-tartrate, Alphagan^{®} (Alphagan is a registered trademark of Allergan, Inc.), and UK14,304.

The term "glaucoma drug" or "second glaucoma drug" encompasses, without limitation, all drugs used to treat glaucoma including those drugs used to lower intraocular pressure with the exception of brimonidine.

The term "histamine antagonist" includes all chemicals that have been found to antagonize at least one histamine receptor including histamine H₁ and histamine H₂.

The term "allergy" or "allergies" or "allergic response" refers to allergic rhinitis and or ocular allergies. Allergic rhinitis is an allergic inflammation of the nasal airways with attendant symptoms, including, but not limited to, rhinorrhea, nasal obstruction, nasal itching, sneezing, ocular pruritis. Ocular allergies are any allergic diseases of the eye, including, but not limited to, seasonal/perennial allergic conjunctivitis, vernal keratoconjunctivitis, giant papillary conjunctivitis, perennial allergic conjunctivitis and atopic keratoconjunctivitis.

The term "pharmaceutically acceptable salts" is meant to include salts of the active compounds which are prepared with relatively nontoxic acids or bases, depending on the particular substituents found on the compounds described herein. When compounds of the present invention contain relatively acidic functionalities, base addition salts can be obtained by contacting the neutral form of such compounds with a sufficient amount of the desired base, either net or in a suitable inert solvent. Examples of pharmaceutically acceptable base addition salts include sodium, potassium, calcium, ammonium, organic amino, or magnesium salt, or a similar salt. When compounds of the present invention contain relatively basic functionalities, acid addition salts can be obtained by contacting the neutral form of such compounds with a sufficient amount of the desired acid, either net or in a suitable inert solvent. Examples of pharmaceutically acceptable acid addition salts include those derived from inorganic acids like hydrochloric, hydrobromic, nitric, carbonic, monohydrogencarbonic, phosphoric, monohydrogenphosphoric, dihydrogenphosphoric, sulfuric, monohydrogensulfuric, hydriodic, or phosphorous acids and the like, as well as the salts derived from relatively nontoxic organic acids like acetic, propionic, isbutyric, oxalic, maleic, malonic, benzoic, succinic, suberic, fumeric mandelic, phthalic, benzenesulfonic, p-tolylsulfonic, citric, tartaric, methanesulfonic, and the like. Also included are salts of amino acids such as arginate and the like, and salts of organic acids like glucuronic or galactunoric acids and the like (see, for example, Berge, S. M., et al., "Pharmaceutical Salts", Journal of Pharmaceutical Science, 1977, 66, 1-19). Certain specific compounds of the present inventions contain both basic and acidic functionalities that allow the compounds to be converted into either base or acid addition salts.

The neutral forms of the compounds may be registered by contacting the salt with a base or acid and isolating the parent compound in the conventional manner. The parent form of the compound differs from the various salt forms in certain physical properties, such as solubility in polar solvents, but otherwise the salts are equivalent to the parent form of the compound for the purposes of the present invention.

In additional to salt forms, the present invention provides compounds which are in a prodrug form. Prodrugs of the compounds described herein are those compounds that readily undergo chemical changes under physiological conditions to provide the compounds of the present invention. Additionally, prodrugs can be converted to the compounds of the present invention by chemical or biochemical methods in an ex vivo environment. For example, prodrugs can be slowly converted to the compounds of the present invention when placed in a transdermal patch reservoir with a suitable enzyme or chemical reagent. Prodrugs are often useful because, in some situations, they may be easier to administer than the parent drug. They may, be bioavailable by oral administration whereas the parent drug is not. The prodrug may also have improved solubility in pharmacological compositions over the parent drug. A wide variety of prodrug derivatives are known in the art, such as those that rely on hydrolytic cleavage or oxidative activation of the prodrug. An example, without limitation, of a prodrug would be a compound of the present invention which is administered as an ester (the "prodrug"), but then is metabolically hydrolyzed to the carboxylic acid, the active entity. Additional examples include peptidyl derivatives of a compound of the invention.

Certain compounds of the present invention can exist in unsolvated forms as well as solvated forms, including hydrated forms. In general, the solvated forms are equivalent to unsolvated forms and are intended to be encompassed within the scope of the present invention. Certain compounds of the present invention may exist in multiple crystalline or amorphous forms. In general, all physical forms are equivalent for the uses contemplated by the present invention and are intended to be within the scope of the present invention.

In preferred embodiments, compositions of the present invention may comprise a nonionic surfactant, a viscosity enhancer, preferably hydroxypropylmethyl cellulose ("HPMC") or carboxymethyl cellulose, a buffer and optionally, sorbate.

Nonionic surfactants suitable for use in the present invention include, but are not limited to a polysorbate, a poloxamer, a polyoxyl, an alkyl aryl poly ether, a cyclodextrin, a tocopheryl polyethylene glycol succinate. a glucosyl dialkyl ethers and a crown ether, ester-linked surfactants. Nonionic surfactants may be present in compositions of the present invention at a concentration from about 1% to about 5% w/v.

Polysorbates suitable for use in the present invention include, but are not limited to, polysorbate 20 (polyoxyethylene (20) sorbitan monolaurate), polysorbate 40 (polyoxyethylene (20) sorbitan monopalmitate), polysorbate 60 (polyoxyethylene (20) sorbitan monostearate) and polysorbate 80 (polyoxyethylene (20) sorbitan monooleate.

Polysorbates may be present in compositions of the present invention at a concentration from about 1% to about 5% w/v, more preferably from about 2% to about 3% w/v and most preferably at about 2.5% w/v.

Cyclodextrins suitable for use in the present invention include, but are not limited to, ionically charged (e.g. anionic) beta - cyclodextrins with or without a butyrated salt (Captisol^{®}) 2-hydroxypropyl beta cyclodextrin ("HPβCD"), alpha cyclodextrins and gamma cyclodextrins.

Poloxamers include but are not limited to poloxamer 103, poloxamer 123, and poloxamer 124, poloxamer 407, poloxamer 188, poloxamer 338 and any poloxamer analogue or derivative

Polyoxyls include but are not limited to Brij^{®} 35, 78, 98, 700 (polyoxyethylene glycol alkyl ethers) and Spans (sorbitan esters) and Span^{®} 20-80 (sorbitan monolaurate, sorbitan monopalmitate, sorbitan monostearate, and sorbitan monooleate).

The viscosity enhancer, preferably HPMC, may be present in compositions of the present invention at a concentration from about 0.1% to about 1.2% w/v, preferably at about 1.2% w/v.

Buffers useful in the present invention include, but are not limited to, citrate buffer and phosphate buffer. Buffers may be present in compositions of the present invention at a concentration from about 1 millimolar ("mM") to about 10 mM, preferably from about 2 mM to about 6 mM and more preferably from about 3 mM to about 4mM.

Sorbate may be present in compositions of the present invention at a concentration from about 0.01% to about 0.5% w/v, preferably from about 0.05% to about 0.25% w/v and more preferably at about 0.1% w/v.

As used herein, all numerical values relating to amounts, weights, and the like, that are defined as "about" each particular value is plus or minus 10%. For example, the phrase "about 5% w/v" is to be understood as "4.5% to 5.5% w/v." Therefore, amounts within 10% of the claimed value are encompassed by the scope of the claims.

As used herein "% w/v" refers to the percent weight of the total composition.

As used herein the terms "subject" and "patient" are used interchangeably and refer, but are not limited to, a person or other animals.

As used herein, the term "prevent" or "preventing" refers to precluding, averting, obviating, forestalling, stopping, or hindering something from happening, especially by advance action.

As used herein, the term "treat" or "treating" refers to reversing, alleviating or slowing the progress of the disease, disorder, or condition to which such terms apply, or one or more symptoms of such disease, disorder, or condition.

As used herein, the term "administration" or "administering" refers to topical application, injection or administration via implants.

The articles "a," "an" and "the" are intended to include the plural as well as the singular, unless the context clearly indicates otherwise.

### Combination with Glaucoma Drugs

It is a discovery of the present invention that brimonidine at concentrations as low as about one-fifth of that in currently available formulations in combination with a second glaucoma drug is capable of greatly reducing intraocular pressure over the second glaucoma drug alone while whitening the eye and greatly reducing and or eliminating the rebound hyperemia associated with brimonidine alone or in fixed combination glaucoma drugs to date. Topical application of this extremely low dose brimonidine ("ELDB") in combination with the second glaucoma drug demonstrates intraocular pressure reduction that is similar to or may be better than full strength brimonidine combinations and may be synergistic over either drug alone.

Further, a major obstacle in treating glaucoma is patient compliance. Many patients stop administering glaucoma therapy due to the attendant side effects including conjunctival hyperemia and rebound hyperemia (i.e. eye redness). It is a discovery of the present invention that the use of ELDB in combination treats glaucoma while greatly reducing side effects such as conjunctival hyperemia associated with full strength brimonidine concentrations. Finally, ELDB is capable of whitening the sclera of the eye beyond the subject's baseline whiteness, which may lead to even greater patient compliance.

Not wishing to be held to a particular theory, ELDB may reduce systemic delivery of the glaucoma drugs and thus reduce systemic side effects and lead to greater retention of the glaucoma drugs on the surface of the eye. This greater retention may lead to greater intraocular absorption of the glaucoma drugs and thus reduce the amount and or frequency of administration, leading to further patient compliance.

In one embodiment, the present invention is directed to an ophthalmological composition for the treatment of glaucoma comprising brimonidine at a concentration from about 0.01% to about 0.050% w/v and a second glaucoma drug.

Brimonidine may be at concentrations from about 0.005% to about 0.050% w/v, preferably from about 0.005% to about 0.050% w/v, from about 0.01% to about 0.050% w/v from about 0.025% to about 0.045% w/v, from about 0.025% to about 0.035% w/v and about 0.025%, about 0.035% or about 0.04% w/v.

Glaucoma drugs, other than brimonidine, that are useful for the present invention include, but are not limited to prostaglandins, rock inhibitors, beta-blockers and carbonic anhydrase inhibitors ("CAI"). Prostaglandins include, but are not limited to, of latanoprost, bimatoprost, travaprost, tafluprost and pharmaceutically acceptable salts thereof. Rho kinase inhibitors include, but are not limited to, netarsudil. Beta-blockers include, but are not limited to, carteolol, timoptic, timolol, betaloxol, levobunolol, metipranolol and pharmaceutically acceptable salts thereof. CAIs included, but are not limited to, brinzolamide, dorzolamide, acetazolamide, methazolamide and pharmaceutically acceptable salts thereof.

Glaucoma drugs other than brimonidine may be at concentrations from about 0.001% to about 5% w/v. Preferably: prostaglandins are at a concentration from about 0.001% to about 0.1% w/v, more preferably from about 0.0015% to about 0.03% w/v; beta-blockers are at a concentration from about 0.1% to about 1% w/v, more preferably from about 0.25% to about 0.5% w/v; rho kinase inhibitors are at a concentration from about 0.01% to about 1% w/v, preferably from about 0.01% to about 0.05% w/v, and CAIs are at a concentration from about 0.5% to about 5% w/v, more preferably from about 1% to about 2% w/v. Glaucoma drugs for ophthalmological administration are currently marketed under the following concentrations of active ingredients: 0.004% travaprost; 0.01% and 0.03% bimatoprost; 0.0015% tafluprost; 0.005% latanoprost; 0.02% netarsudil; 0.25% and 0.5% timolol maleate; 0.25% and 0.5% timolol hemihydrate; 0.25% and 0.5% betaxolol hydrochloride ("HCl"); 0.25% and 0.5% levobunolol HCl; 1% brinzolamide; and 2% dorzolamide.

Specific combinations of brimonidine and a second glaucoma drug include, but are not limited to: about 0.035% to 0.050% w/v brimonidine and about 0.005% w/v latanoprost; about 0.035% to 0.050% w/v brimonidine and about 0.050% w/v bimatoprost; about 0.035% to about 0.050% w/v brimonidine and about 0.5% w/v timolol; and about 0.035% to about 0.050% w/v brimonidine and about 2% w/v dorzolamide.

Compositions of the present invention may have a pH from about 5.0 to about 8.0, more preferably from about.6.0 to about 7.5, even more preferably from about 7.2 to about 7.7 for brimonidine glaucoma combination drugs and even more preferably from about 7.4 to about 7.6.

In a preferred embodiment, the present invention is directed to a composition comprising:
about 0.001% w/v latanoprost;
about 0.050% w/v brimonidine;
about 2.5% w/v polysorbate;
about 1.2% w/v hydroxypropylmethyl cellulose ("HPMC");
about 5 mM boric acid; and
optionally, about 0.1% w/v sorbate,
wherein the composition has a pH of about 7.4.

Methocell^{®} was used as the source of HPMC (Methocell is a registered trademark of and available from Dow Corning).

In another preferred embodiment, the present invention is directed to a composition comprising:
about 0.05% w/v timolol;
about 0.050% w/v brimonidine;
about 2.5% w/v polysorbate;
about 1.2% w/v HPMC;
about 4 mM boric acid; and
optionally, about 0.1% w/v sorbate,
wherein the composition has a pH of about 7.4.

Compositions of the present invention for the treatment of glaucoma may be administered topically to the eye, via intraocular injection or via intraocular implant, preferably administration occurs via topical application.

### Combination with Histamine Antagonists

Currently available anti-histamines (i.e. histamine antagonists) are not capable of fully alleviating all allergy symptoms including particularly the eye redness associated with cytokine induced vasodilation. It is a discovery of the present invention that the use of extremely low dose brimonidine ("ELDB") in combination with an anti-histamine leads to not only greater reduction of redness but greater alleviation of allergy symptoms such as conjunctival swelling than the use of the anti-histamine alone.

In one embodiment, the present invention is directed to an ophthalmological composition for the treatment of allergies comprising brimonidine at a concentration from about 0.005% to about 0.050% w/v and a histamine antagonist.

Brimonidine may be at concentrations from about 0.005% to about 0.050% w/v, preferably from about 0.01% to about 0.050% w/v, more preferably from about 0.025% to about 0.035% w/v and about 0.025%, about 0.035% or about 0.04% w/v.

Histamine antagonist useful for the present invention include, but are not limited to naphazoline, antazoline, azelastine, carbinoxamine, cyproheptadine, emedastine, hydroxyzine, levocabastine, brompheniramine, chlorpheniramine, clemastine, diphenhydramine, ketotifen, loratadine, desloratadine, cetirizine, fexofenadine, olopatadine, acrivastine, ebastine, norastemizole, levocetirizine, mizolastine, pheniramine, pharmaceutically acceptable salts thereof and combinations thereof.

Histamine antagonist may be at concentrations from about 0.01% to about 1% w/v, preferably from about 0.025% to about 0.7% w/v. Histamine antagonists for nasal or ophthalmological administration are currently marketed under the following concentrations of active ingredients: 0.05% naphazoline HCl; combination of 0.05% naphazoline HCl and 0.5% antazoline phosphate; combination of 0.05% naphazoline HCl and 0.3% pheniramine maleate; combination of 0.02675% naphazoline hydrochloride and 0.315% pheniramine maleate; 0.1% and 0.15% azelastine; 0.05% emedastine; 0.05% levocabastine HCl; 0.025% ketotifen; and 0.1%, 0.2% and 0.7% olopatadine.

In a preferred embodiment, the present invention is directed to a composition comprising:
about 0.0035% w/v ketotifen fumarate;
about 0.035% w/v brimonidine;
about 2.5% w/v polysorbate;
from about 0.1% to about 1.2% w/v HPMC;
about 4 mM citrate buffer; and
optionally, about 0.1% w/v sorbate,
wherein the composition has a pH of 6.5.

### Combination with Non-steroidal Anti-Inflammatory Drugs

Currently available non-steroidal anti-inflammatory drugs ("NSAIDs") are not capable of fully reducing all inflammation. It is a discovery of the present invention that the use of extremely low dose brimonidine ("ELDB") in combination with an NSAID leads to greater reduction of inflammation than the use of the an NSAID alone. Further, there is considerable inflammation, hyperemia and irritation largely known to be cytokine induced, where a combination of low dose brimonidine and low strength NSAID, such as ketorolac 0.025%, can provide both relief of hyperemia, reduction of inflammation. It is believed the combination can improve the quality of life for one suffering from chronic ocular allergies better than either product alone.

In one embodiment, the present invention is directed to an ophthalmological composition for the reduction of inflammation comprising brimonidine at a concentration from about 0.005% to about 0.050% w/v and an NSAID.

Brimonidine may be at concentrations from about 0.005% to about 0.050% w/v, preferably from about 0.01% to about 0.050% w/v, more preferably from about 0.025% to about 0.035% w/v and about 0.025%, about 0.035% or about 0.04% w/v.

NSAIDs useful for the present invention include, but are not limited to ketorolac, aspirin, celecoxib, diflunisal, etodolac, ibuprofen, indomethacin, ketoprofen, nabumetone, naproxen, oxaprozin, salsalate, sulindac, tolmetin and pharmaceutically acceptable salts thereof and combinations thereof.

NSAIDs may be at concentrations from about 0.01% to about 10% w/v, preferably from about 0.01% to about 1% w/v, more preferably from about 0.02% to about 0.50% w/v.

Compositions of the present invention for the reduction of inflammation may be administered topically to the eye or nasal cavity via drops or spray.

### EXAMPLES

### Example 1-Prophetic Intraocular Pressure Reduction

### Method

A subject with normo-tensive baseline intraocular pressure ("IOP"; < 21 mm Hg) was prophetically treated for 5 consecutive days with a single instillation of two drops per eye per day of a composition containing 0.035% brimonidine, 0.005% latanoprost, and a combination of 0.03% brimonidine and 0.005% latanoprost. A 1-week washout period was observed between each of the three treatment cycles. Instillation was performed at 8:30 AM, followed by 30 seconds of punctual occlusion with instillation on days 1, 3, and 5.

IOP was measured at one or more of 4 hrs, 8 hrs, 12 hrs, 24 hrs, 32 hrs and comfort and side effect profile were qualitatively assessed.

### Results

Based on preliminary data, the tested inventive formulations prophetically achieved greater IOP reduction than latanoprost alone. Further, a peak IOP reduction effect is prophetically achieved at about 4 to 8 hours after instillation and the IOP remained below the baseline 24 hours after instillation. Figure 1 demonstrates a prophetic example of IOP reduction after administration of 0.005% latanoprost (0 hours, white bar), versus IOP reduction after administration of 0.03% brimonidine and 0.005% latanoprost (4, 6 and 8 hours, black bar). In fact, the reduction in IOP after administration of the combination of ELDB and latanoprost prophetically achieved synergy at all times and days except prior to instillation. Synergy will be calculated by taking the observed reduction in IOP and dividing by the expected reduction in IOP to give a "synergy factor". Synergy factors greater than 1 demonstrate synergy. Expected IOP reduction will be calculated using the following formula where A is the IOP reduction for ELDB alone and B is the IOP reduction for latanoprost alone: A + B - (AB/100). Thus, the formulations of the invention prophetically demonstrate improved performance over full strength brimonidine and latanoprost alone under similar conditions of testing. Finally, during testing of latanoprost alone subject prophetically experienced ocular hyperemia that was not observed during the testing of brimonidine or the brimonidine and latanoprost combination demonstrating that extremely low dose brimonidine is capable of not only enhancing IOP reduction but also reducing attendant side effects of glaucoma drugs.

In conclusion, the combination of ELDB and a second glaucoma drug prophetically provide enhanced and synergistic relief of intraocular pressure that is either as good or better than that provided by the second glaucoma drug alone or the second glaucoma drug in combination with full strength brimonidine. Further, the combination of ELDB and the second glaucoma drug prophetically provide reduced or ameliorated side effects that normally occur when the second glaucoma drug is administered alone.

### Example 2-Prophetic Allergy Relief

### Method

A subject suffering from nasal allergies was prophetically administered a combination of 0.005% to 0.050% w/v brimonidine and either 0.025% to 0.035% w/v ketotifen fumarate or 0.3% w/v pheniramine maleate for 7 consecutive days.

### Result

The tested inventive formulations prophetically achieved greater reduction in cytokines and inflammation than ketotifen or pheniramine alone. Further, the tested inventive formulations prophetically reduced eye redness associated with ketotifen and pheniramine. Finally, the tested inventive formulations prophetically whitened eyes of the subject beyond the baseline whiteness.

### EMBODIMENTS

Embodiment 1. An ophthalmological composition for the treatment of glaucoma comprising from about 0.01% to about 0.050% w/v brimonidine and an effective amount of a second glaucoma drug, wherein w/v denotes weight by total volume of the composition.

Embodiment 2. The composition of embodiment 1, wherein the brimonidine is at a concentration from about 0.035% to about 0.050% w/v.

Embodiment 3. The composition of embodiment 1, wherein the second glaucoma drug is selected from the group consisting of a prostaglandin, a beta-blocker, a rho kinase inhibitor, and a carbonic anhydrase inhibitor.

Embodiment 4. The composition of embodiment 1, wherein the glaucoma drug is selected from the group consisting of latanoprost, bimatoprost, travaprost, tafluprost, netarsudil, carteolol, timoptic, timolol, betaloxol, levobunolol, metipranolol, brinzolamide, dorzolamide, acetazolamide, methazolamide and pharmaceutically acceptable salts thereof.

Embodiment 5. The composition of embodiment 1, wherein the second glaucoma drug is at a concentration from about 0.001% to about 1.0% w/v.

Embodiment 6. The composition of embodiment 3, wherein the second glaucoma drug is a prostaglandin at a concentration from about 0.001% to about 0.1% w/v or a rho kinase inhibitor at a concentration from about 0.01% to about 1.0% w/v or a beta-blocker at a concentration from about 0.1% to about 1.0% w/v or a carbonic anhydrase inhibitor at a concentration from about 0.5% to about 5% w/v.

Embodiment 7. The composition of embodiment 3, wherein the second glaucoma drug is a prostaglandin at a concentration from about 0.0015% to about 0.03% w/v, a rho kinase inhibitor at a concentration from about 0.01% to about 0.05% w/v, beta-blocker at a concentration from about 0.25% to about 0.5% w/v or a carbonic anhydrase inhibitor at a concentration from about 1% to about 2% w/v.

Embodiment 8. The composition of embodiment 1, wherein brimonidine is at a concentration of about 0.050% w/v and the second glaucoma drug is latanoprost at a concentration of about 0.005% w/v.

Embodiment 9. The composition of embodiment 1, wherein brimonidine is at a concentration of about 0.050% w/v and the second glaucoma drug is bimatoprost at a concentration of about 0.05% w/v.

Embodiment 10. The composition of embodiment 1, wherein brimonidine is at a concentration of about 0.050% w/v and the second glaucoma drug is timolol at a concentration of about 0.5% w/v.

Embodiment 11. The composition of embodiment 1, wherein brimonidine is at a concentration of about 0.04% w/v and the second glaucoma drug is dorzolamide at a concentration of about 2% w/v.

Embodiment 12. The composition of embodiment 1, further comprising a nonionic surfactant, a viscosity enhancer, a buffer and optionally, sorbate.

Embodiment 13. The composition of embodiment 12, wherein the nonionic surfactant is a polysorbate at a concentration from about 1% to about 5% w/v.

Embodiment 14. A method of treating glaucoma comprising administering the composition of embodiment 1 to a subject in need thereof.

Embodiment 15. The method of embodiment 14, wherein the method further provides redness reduction.

Embodiment 16. The method of embodiment 14, wherein the method further provides eye whitening.

Embodiment 17. The method of embodiment 14, wherein the method further provides conjunctival swelling reduction, hyperemia reduction, eye whitening or a combination thereof.

Embodiment 18. An ophthalmological composition for the treatment of allergies comprising from about 0.005% to about 0.050% brimonidine and an effective amount of a histamine antagonist, wherein w/v denotes weight by total volume of the composition.

Embodiment 19. The composition of embodiment 18, wherein the brimonidine is at a concentration from about 0.025% to about 0.035% w/v.

Embodiment 20. The composition of embodiment 18, wherein the histamine antagonist is selected from the group consisting of naphazoline, antazoline, azelastine, carbinoxamine, cyproheptadine, emedastine, hydroxyzine, levocabastine, brompheniramine, chlorpheniramine, clemastine, diphenhydramine, ketotifen, loratadine, desloratadine, cetirizine, fexofenadine, olopatadine, acrivastine, ebastine, norastemizole, levocetirizine, mizolastine, pheniramine, pharmaceutically acceptable salts thereof and combinations thereof.

Embodiment 21. The composition of embodiment 18, wherein the histamine antagonist is at a concentration from about 0.025% to about 0.7% w/v.

Embodiment 22. The composition of embodiment 18, further comprising a nonionic surfactant, hydroxypropylmethyl cellulose, a buffer and optionally, sorbate.

Embodiment 23. The composition of embodiment 22, wherein the nonionic surfactant is a polysorbate at a concentration from about 1% to about 5% w/v.

Embodiment 24. A method of treating or preventing an allergic response comprising administering the composition of embodiment 16 to a subject in need thereof.

Embodiment 25. The method of embodiment 24, wherein the method further provides eye whitening.

Embodiment 26. The method of embodiment 24, wherein the method further provides selective vasoconstriction of smaller vessels, reduction of postcapillary venule leakage, reduction of inflammation from cytokines, leukotrienes or a combination thereof.

Embodiment 27. An ophthalmological composition for reducing of inflammation comprising from about 0.005% to about 0.050% w/v brimonidine and an effective amount of a nonsteroidal anti-inflammatory drug (NSAID), wherein w/v denotes weight by total volume of the composition.

Embodiment 28. The composition of embodiment 27, wherein the brimonidine is at a concentration from about 0.025% to about 0.035% w/v.

Embodiment 29. The composition of embodiment 27, wherein the NSAID is selected from the group consisting of ketorolac, aspirin, celecoxib, diflunisal, etodolac, ibuprofen, indomethacin, ketoprofen, nabumetone, naproxen, oxaprozin, salsalate, sulindac, tolmetin and pharmaceutically acceptable salts thereof and combinations thereof.

Embodiment 30. The composition of embodiment 29, wherein the NSAID is ketorolac. Embodiment 31. The composition of embodiment 27, wherein the NSAID is at a concentration from about 0.02% to about 0.50% w/v.

Embodiment 32. The composition of embodiment 27, further comprising a nonionic surfactant, a viscosity enhancer, a buffer and optionally, sorbate.

Embodiment 33. The composition of embodiment 32, wherein the nonionic surfactant is a polysorbate at a concentration from about 1% to about 5% w/v.

Embodiment 34. A method of reducing inflammation comprising administering the composition of embodiment 25 to a subject in need thereof.

Embodiment 35. A composition for the treatment of glaucoma comprising:
about 0.001% w/v latanoprost or 0.05% w/v timolol;
about 0.050% w/v brimonidine;
about 2.5% w/v polysorbate;
about 1.2% w/v hydroxypropylmethyl cellulose;
about 5 millimolar boric acid; and
optionally, about 0.1% w/v sorbate,
wherein the composition has a pH of about 7.4.

Embodiment 36. A composition for the treatment of allergic conjunctivitis comprising:
about 0.0035% w/v ketotifen fumarate;
about 0.035% w/v brimonidine;
about 2.5% w/v polysorbate;
from about 0.1% to about 1.2% w/v hydroxypropylmethyl cellulose;
about 4 millimolar citrate buffer; and
optionally, about 0.1% w/v sorbate,
wherein the composition has a pH of about 6.5.

## Claims

1. An ophthalmological composition for the treatment of allergies comprising from about 0.005% to about 0.050% brimonidine and an effective amount of a histamine antagonist, wherein w/v denotes weight by total volume of the composition.

2. The composition of claim 1, wherein the brimonidine is at a concentration from about 0.025% to about 0.035% w/v.

3. The composition of claim 1, wherein the histamine antagonist is selected from the group consisting of naphazoline, antazoline, azelastine, carbinoxamine, cyproheptadine, emedastine, hydroxyzine, levocabastine, brompheniramine, chlorpheniramine, clemastine, diphenhydramine, ketotifen, loratadine, desloratadine, cetirizine, fexofenadine, olopatadine, acrivastine, ebastine, norastemizole, levocetirizine, mizolastine, pheniramine, pharmaceutically acceptable salts thereof and combinations thereof.

4. The composition of claim 1, wherein the histamine antagonist is at a concentration from about 0.025% to about 0.7% w/v.

5. The composition of claim 1, further comprising a nonionic surfactant, hydroxypropylmethyl cellulose, a buffer and optionally, sorbate.

6. The composition of claim 5, wherein the nonionic surfactant is a polysorbate at a concentration from about 1% to about 5% w/v.

7. The composition of claim 1 for use in a method of treating or preventing an allergic response comprising administering the composition of claim 1 to a subject in need thereof.

8. The composition for use in the method of claim 7, wherein the method further provides eye whitening.

9. The composition for use in the method of claim 7, wherein the method further provides selective vasoconstriction of smaller vessels, reduction of postcapillary venule leakage, reduction of inflammation from cytokines, leukotrienes or a combination thereof.

10. A composition for the treatment of allergic conjunctivitis comprising:
about 0.0035% w/v ketotifen fumarate;
about 0.035% w/v brimonidine;
about 2.5% w/v polysorbate;
from about 0.1% to about 1.2% w/v hydroxypropylmethyl cellulose;
about 4 millimolar citrate buffer; and
optionally, about 0.1% w/v sorbate,
wherein the composition has a pH of about 6.5.

11. An ophthalmological composition for the treatment of glaucoma comprising from about 0.01% to about 0.050% w/v brimonidine and an effective amount of a second glaucoma drug, wherein w/v denotes weight by total volume of the composition.

12. The composition of claim 11, wherein the brimonidine is at a concentration from about 0.035% to about 0.050% w/v.

13. The composition of claim 11, wherein the second glaucoma drug is selected from the group consisting of a prostaglandin, a beta-blocker, a rho kinase inhibitor, and a carbonic anhydrase inhibitor.

14. The composition of claim 11, wherein the glaucoma drug is selected from the group consisting of latanoprost, bimatoprost, travaprost, tafluprost, netarsudil, carteolol, timoptic, timolol, betaloxol, levobunolol, metipranolol, brinzolamide, dorzolamide, acetazolamide, methazolamide and pharmaceutically acceptable salts thereof.

15. The composition of claim 11, wherein the second glaucoma drug is at a concentration from about 0.001% to about 1.0% w/v.

16. The composition of claim 13, wherein the second glaucoma drug is a prostaglandin at a concentration from about 0.001% to about 0.1% w/v or a rho kinase inhibitor at a concentration from about 0.01% to about 1.0% w/v or a beta-blocker at a concentration from about 0.1% to about 1.0% w/v or a carbonic anhydrase inhibitor at a concentration from about 0.5% to about 5% w/v.

17. The composition of claim 13, wherein the second glaucoma drug is a prostaglandin at a concentration from about 0.0015% to about 0.03% w/v, a rho kinase inhibitor at a concentration from about 0.01% to about 0.05% w/v, beta-blocker at a concentration from about 0.25% to about 0.5% w/v or a carbonic anhydrase inhibitor at a concentration from about 1% to about 2% w/v.

18. The composition of claim 11, wherein brimonidine is at a concentration of about 0.050% w/v and the second glaucoma drug is latanoprost at a concentration of about 0.005% w/v.

19. The composition of claim 11, wherein brimonidine is at a concentration of about 0.050% w/v and the second glaucoma drug is bimatoprost at a concentration of about 0.05% w/v.

20. The composition of claim 11, wherein brimonidine is at a concentration of about 0.050% w/v and the second glaucoma drug is timolol at a concentration of about 0.5% w/v.

21. The composition of claim 11, wherein brimonidine is at a concentration of about 0.04% w/v and the second glaucoma drug is dorzolamide at a concentration of about 2% w/v.

22. The composition of claim 11, further comprising a nonionic surfactant, a viscosity enhancer, a buffer and optionally, sorbate.

23. The composition of claim 22, wherein the nonionic surfactant is a polysorbate at a concentration from about 1% to about 5% w/v.

24. The composition of claim 11 for use in a method of treating glaucoma comprising administering the composition of claim 11 to a subject in need thereof.

25. The composition for use in the method of claim 24, wherein the method further provides redness reduction.

26. The composition for use in the method of claim 24, wherein the method further provides eye whitening.

27. The composition for use in the method of claim 24, wherein the method further provides conjunctival swelling reduction, hyperemia reduction, eye whitening or a combination thereof.

28. An ophthalmological composition for reducing of inflammation comprising from about 0.005% to about 0.050% w/v brimonidine and an effective amount of a nonsteroidal anti-inflammatory drug (NSAID), wherein w/v denotes weight by total volume of the composition.

29. The composition of claim 28, wherein the brimonidine is at a concentration from about 0.025% to about 0.035% w/v.

30. The composition of claim 28, wherein the NSAID is selected from the group consisting of ketorolac, aspirin, celecoxib, diflunisal, etodolac, ibuprofen, indomethacin, ketoprofen, nabumetone, naproxen, oxaprozin, salsalate, sulindac, tolmetin and pharmaceutically acceptable salts thereof and combinations thereof.

31. The composition of claim 30, wherein the NSAID is ketorolac.

32. The composition of claim 28, wherein the NSAID is at a concentration from about 0.02% to about 0.50% w/v.

33. The composition of claim 28, further comprising a nonionic surfactant, a viscosity enhancer, a buffer and optionally, sorbate.

34. The composition of claim 33, wherein the nonionic surfactant is a polysorbate at a concentration from about 1% to about 5% w/v.

35. The composition of claim 28 for use in a method of reducing inflammation comprising administering the composition of claim 28 to a subject in need thereof.

36. A composition for the treatment of glaucoma comprising:
about 0.001% w/v latanoprost or 0.05% w/v timolol;
about 0.050% w/v brimonidine;
about 2.5% w/v polysorbate;
about 1.2% w/v hydroxypropylmethyl cellulose;
about 5 millimolar boric acid; and
optionally, about 0.1% w/v sorbate,
wherein the composition has a pH of about 7.4.
